# EUROPEAN PATENT APPLICATION

(11) **EP 2 258 374 A2**
(43) Date of publication of application: **08.12.2010**
(21) Application number: 09728965.6
(22) Date of filing: 03.04.2009
(51) Int. Cl.: A61K 31/56, A61P 19/02

(54) **USE OF MASLINIC ACID FOR THE TREATMENT OF DISEASES AND THE SYMPTOMS THEREOF BY MEANS OF COX-2 INHIBITION**

(30) Priority: 03.04.2008 ES 200801069
(71) Applicant: Biomaslinic S.L., 18100 Armilla (Granada) (ES); Universidad De Granada, 18100 Armilla (Granada) (ES)
(72) Inventor: PRADOS OSUNA, José, 18100 Armilla (Granada) (ES); GARCÍA-GRANADOS LÓPEZ DE HIERRO, Andrés, 18100 Armilla (Granada) (ES); PARRA SÁNCHEZ, Andrés, 18100 Armilla (Granada) (ES); MARTÍNEZ RODRÍGUEZ, Antonio, 18100 Armilla (Granada) (ES)
(74) Representative: Carvajal y Urquijo, Isabel
(86) International application number: PCT/ES2009/000195
(87) International publication number: WO 2009/121992

(57) **Abstract**

The invention relates to the use of maslinic acid or natural, synthetic or semi-synthetic maslinic-rich mixtures or of a composition containing said acid for the treatment of pathological processes associated with COX-2 activation, intended inter alia, for the symptomatic and/or regenerative treatment of arthrosis, rheumatoid arthritis, fibromyalgia, sciatica and other articular disorders that are difficult to diagnose. The invention also relates to the topical administration thereof.

## Description

The present invention refers to the use of maslinic acid and any of its derivates for the treatment of pathologic processes comprising the inhibition of endogenous cyclooxygenase-2 (COX-2). More specifically for the symptomatic and/or asymptomatic treatment of arthrosis, rheumatoid arthritis, fibromyalgia, sciatica and other articular discomforts. It also refers to the topical administration of a composition, which comprises maslinic acid, any of its derivates, or natural, synthetic or semisynthetic mixtures rich in maslinic or its derivates.

### STATE OF THE ART

The oleanolic acid (3-beta-Hydroxy-12-oleanen-28-oic acid) is a triterpenic acid ubiquitously distributed in the vegetal kingdom. Thus, the phytochemical database of the United States Department of Agriculture (USDA) registers its presence in almost one hundred plants, among which we can find the Olea europaea, as well as a series of verified biological activities (antiabortion, anticariogenic, antifertility, antihepatotoxic, anti-inflammatory, antisarcomic, cancer preventive, cardiotonic, diuretic, hepatoprotector and uterotonic).

The maslinic acid (2- alpha,3- beta-dihydroxyolean-12-en-28-oic acid), also called crataegolic acid, is a much less distributed acid in nature, having been detected in some ten plants. Its activity as antihistaminic and anti-inflammatory is known, although its scarcity causes that it has not been studied thoroughly. The isolation of the oleanolic and maslinic acids of the waxes of the surface of the Olea europaea fruit has been described [Bianchi, G., Pozzi, N. And Vlahov, G. Phytochemistry (1994) 37, 205-207] through the methanolic extraction of olives previously washed with chloroform. The separation of this type of acids has been described by High Speed Countercurrent Chromatography (HSCCC) [Du, Q.Z., Xiong, X.P. and Ito, Y.; Journal of Liquid Chromatography (1995) 18, 1997-2004].

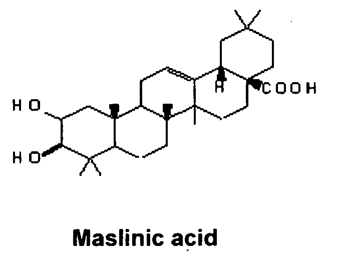

Nowadays, there exist a great number of patents where maslinic acid acts as an active component: antitumor agent US2003153538 or WO0252956; apoptosis inducer WO03057224; anorectic food WO203039270 and WO03011267; external and bleaching agent for the skin US2003133958; vascular lesions WO02078685.

In some publications it is suggested that this triterpene can also have anti-inflammatory activity (Marquez-Martin A, De La Puerta R, Fernandez-Arche A, Ruiz-Gutierrez V, Yaqoob P. Cytokine. (2006) Dec; 36 (5-6):211-7). In this document it is also described that the maslinic acid inhibits the release of IL-6 and TNFα and the production of NO and of the hydrogen peroxide (ROS) in LPS-stimulated murine peritoneal macrophages, an effect which the authors do not find in mononuclear cells of human peripheral blood. In any case, what these discrepancies clearly show is that the answers are not homogeneous and therefore, they are not predictable, which makes necessary the study of each model and/or type of tissue.

On the other hand, the prostaglandins are synthesized from the arachidonic acid (and of other unsaturated fatty acids C20 called eicosanoids) following the metabolic pathway of cyclooxygenase (COX), a widely distributed enzyme which can produce a cycle and introduce oxygen in the precursors to create the prostaglandins all of which derive prostanoic acid. There exist two well-identified different forms of cyclooxygenase which are COX-1 and COX-2. COX-1 is expressed constitutively in many tissues, where it regulates important physiological functions. For example, COX-1 catalyzes the production of prostaglandins by the endothelium and the gastric mucosa, which leads to antithrombogenic and cytoprotective effects, respectively. COX-2, which is the inducible form, is undetectable in most tissues. COX-2 is a specific isoform derived from a different gene to the one that codifies COX-1. The expression of COX-2 increases during the development and inflammation, but its constitutive expression remains low in most organs, except for the brain.

Prostaglandins, particularly PGE₂, fulfill multiple physiological and physiopathological functions. Thus, low concentrations of PGE₂ keep the integrity of the normal gastric mucosa and the homeostasis of fluids and electrolytes while the levels of this mediator are implied in inflammatory processes.

Other metabolites of arachidonic acid are isoprostanes. These metabolites are structurally similar to prostaglandins and they are generated by the oxidation of fatty acids of the plasmatic membrane.

Nowadays, it is widely known that isoprostanes represent one of the best cell and systematic markers of oxidative stress. As it has been shown the antioxidant activity of maslinic acid it is possible that this compound can inhibit the formation of isoprostanes (Marquez-Martin A, De La Puerta R, Fernandez-Arche A, Ruiz-Gutierrez V, Yaqoob P. Cytokine. (2006) Dec; 36(5-6):211-7); Montilla MP, Agil A, Navarro MC, Jimenez MI, Garcia-Granados A, Parra A, Cabo MM. Planta Med. (2003) May;69(5):472-4).

According to the University of Maryland Medical Center regarding treatment for rheumatoid arthritis, this treatment can be made, basically, with COX-2 inhibitors, indicating that the "COX-2 inhibitors block an enzyme which favours inflammation, called COX-2" but they do not contemplate efficient treatments for topical administration. Initially, it was thought that this type of drugs worked as well as Nonsteroidal Anti-inflammatory Drugs (NSAIDs), but with fewer stomach problems. However, numerous reports of heart attacks and strokes have led FDA to re-evaluate the risk-benefit relationship of COX-2. Rofecoxib (Vioxx®) and valdecoxib (Bextra®) have been removed from the American market after reports of heart attacks in patients who were taking them. Celecoxib (Celebrex®) is still available, but with a strong warning label and with the recommendation that it be prescribed in the smallest dose and during the shortest time possible. Patients must ask their doctor if this drug is appropriate and safe for them.

Antimalarial drugs such as hydroxychloroquine (Plaquenil®) and sulfasalazine (Azulfidine®) are also beneficial generally joined with methotrexate. But it can take weeks or months before the benefits of this drug become manifest and as they are associated with toxic side effects, it is imperative to be submitted to frequent blood tests while these drugs are in use.

Tumor necrosis factor (TNF) inhibitors are a type of relatively new drugs which are used to treat an autoimmune disorder and, among others, they are: etanercept (Enbrel®), infliximab (Remicade®) and adalimumab (Humira®). Adalimumab® y Etanercept® are injectable drugs, while Infliximab is administered intravenously.

Another relatively new drug is injectable anakinra (Kineret®), an artificial protein which blocks the inflammatory protein interleukin-1. This drug is used to reduce the progression from moderate to serious of active rheumatoid arthritis in patients over 18 who have not responded to one or more of the DMARD (Disease-modifying antirheumatic drugs). Kineret® can be used with another DMARD or with Tumor necrosis factor inhibitors.

Other drugs that suppress the immune system, such as azathioprine (Imuran®) and cyclophosphamide (Cytoxan®), are sometimes used in people who have had no success with other therapies. These drugs, which are associated to toxic side effects, are generally reserved for serious cases of rheumatoid arthritis.

It is unknown the existence of an effective and easy-to-administer treatment for the symptomatic and/or regenerative treatment of arthrosis, rheumatoid arthritis or other hard to diagnose articular problems which acts directly on chondrocytes and achieves an effective treatment with rapid and prolonged disappearance of the indicated symptoms.

### DESCRIPTION OF THE INVENTION

The present invention describes the use of maslinic acid and any of its derivates for the treatment of any pathology comprising the activation of the cyclooxygenase-2 (COX-2) enzyme.

In this sense, in the present invention it is shown the activity of the maslinic acid as inhibitor of the COX-2 activity induced in human chondrocytes and therefore the usefulness of this acid in the treatment of diseases comprising the activation of said COX-2 enzyme.

It is also shown that maslinic acid is capable not only of reducing the inflammation, associated to this type of diseases, but it also intervenes in the reduction or disappearance of symptoms and in chondrocyte tissue regeneration showing long-lasting effects.

As a result of the aforementioned, the invention refers to the symptomatic and/or regenerative treatment of diseases such as arthrosis, rheumatoid arthritis, fibromyalgia, sciatica, jumper syndrome, protuberance of the metatarsophalangeal joint (bunion) and processes that respond to NSAIDs (Nonsteroidal Anti-inflammatory Drugs) specific for COX-2, with a composition comprising maslinic acid or any of its derivatives. This treatment is carried out with a simple administration and it is effective since, among other things, it acts directly on the chondrocytes or affected tissue. Particularly, it is shown in the present invention the effectiveness of the symptomatic and/or regenerative treatment of arthrosis and rheumatoid arthritis with the fast and prolonged disappearance of symptoms.

In the present invention, it is shown the effectiveness of the treatment even with topical administration. It is important to highlight this fact, since other NSAIDs inhibitors of COX-2 do not show effectiveness when they are topically administered and besides, their administration through other ways has serious side effects.

Maslinic acid can be obtained naturally or synthetically. The compositions comprising this maslinic acid include in their definition the use of plant extracts containing maslinic acid, extracts from the by-products of industrial processing of the plant or its fruits or an industrial by-product of the plant containing a higher concentration of maslinic acid than the originally present in the plant. For example, but without limiting to, plant extracts of *Olea* genus, more particularly the *O. europea* species. Besides this type of maslinic acid, its derivatives can also be used, wherein derivates in the present invention refer to their salts or derivatives accepted in pharmacopoeia which facilitate their absorption and transport in the tissues to be treated. More particularly, their sodium, potassic salts or salts from biologically acceptable amines.

These facts are shown through the following experiences carried out:
- It was determined the effect of maslinic acid and its derivatives on the expression of COX-2 protein in primary human monocytes of peripheral blood.
- It was determined the effect of maslinic acid and its derivatives on the enzymatic activity of COX-2 in monocytes of peripheral blood.
- It was determined the effect of maslinic acid and its derivatives on the formation of isoprostanes in primary human monocytes of peripheral blood.
- It was determined the effect of maslinic acid and its derivatives on the release of PGE2 in primary human chondrocyte.
- It was determined the effect of maslinic acid and its derivatives on the release of PGE2 in primary rat astrocytes and in the SK-S-NH neuroblastoma type cell line.
- It was determined the effect of maslinic acid and its derivatives on the transcriptional regulation of the COX-2 gene and on the expression of COX-2 protein in primary astrocytes.
- Clinical tests have been carried out on patients suffering from atrophic, arthrous, arthritic, fibromyalgic processes or tendinosis, among others, to prove the effectiveness of the "in vivo" treatment and more particularly by topical administration.

It is necessary to take into account that, sometimes, previous diagnoses given to the treated patients are not very precise, sometimes being the symptoms the only medium used for the aforementioned diagnosis.

Since the functionality of COX-2 and the production of PEG2 is regulated at several levels and maslinic acid inhibits the production of PGE2 in primary human monocytes it was studied in detail the effect of this triterpene on the expression and function of COX-2 in different "in vitro" cell types as a previous step before the studies with specific animal models. Thus, for the first time it was shown the "in vitro" action of maslinic acid in the inhibition of COX-2 induced in human chondrocytes and a thorough clinical study of patients with arthrosis, arthritis or fibromyalgia, among others, was carried out, showing a surprising effectiveness of the maslinic acid and its salts even in simple topical treatments of the areas showing these syndromes. In fact, although in all the patients with arthrosis an improvement of symptoms was observed, in those younger than 60 years old, there was a completely remission of symptoms in less than one month of treatment. Besides, in all cases, it is observed in a very symptomatic way the gradual reabsorption of calcifications typical of arthrous syndromes.

In more than 80% of cases the effectiveness regarding symptoms starts to be noticeable in 24/48 hours and between 2 and 6 topical applications. Besides the improvement of symptoms in joints (pain and stiffness) patients with evident inflammation, for example in their fingers, notice in few minutes a temperature increase in the treated areas, which clearly indicates an increase of blood circulation the patient starts to experience immediately.

It was proved that maslinic acid is a powerful inmunomodulator of the immune answer since it inhibits the activation and proliferation of T lymphocytes stimulated via TCR and inhibits the production of proinflammatory cytokines TNFα and IL-6, besides the production of prostaglandin E2 (PGE2) in primary monocytes. Also, it is observed that the maslinic acid concentrations which act inhibiting the innate and acquired immune responses are noticeably lower than those required to induce apoptosis in tumor cell lines.

In the present invention, and as a consequence of the results obtained, shown in the example section, it is shown that:
1. Maslinic acid inhibits the production of PGE2 in human monocytes.
2. Maslinic acid inhibits the enzymatic activity of COX-2.
3. Maslinic acid does not inhibit the transcriptional activation of the gene promoter of COX-2 nor the protein expression.
4. Maslinic acid at high doses inhibits the production of PGE2 in chondrocytes and in cells derived from a neuroblastoma.
5. Maslinic acid inhibits the production of isoprostanes in human monocytes.
6. Maslinic acid inhibits the production of IL-6 in human monocytes and chondrocytes.
7. The inhibition of the production of IL-6 by MA is not produced by a transcriptional inhibition of the gene of IL-6.

It has to be taken into account that the junctions between the bones are covered by the articular cartilage, a tissue that does not contain capillaries or nerve endings. During the articular movement the friction between bones is minimized by this articular cartilage, which contains chondrocytes, which represent the 1-2% of the total volume, and whose function is synthetizing proteins of the extracellular matrix. This matrix contains a 70-80% of water which enables to have a structure that serves to mitigate pressures. The electrostatic interaction between water molecules and sulfated mucopolysaccharides is necessary to maintain the hydration of the cartilage. Therefore, proteoglycans lubricated by hyaluronic polymers are the substance that maintains the cartilage integrity. Glycosaminoglycans and proteoglycans maintain an intimate relation with the collagen fibers produced by chondrocytes, which also produce proteoglycans.

For all of this, a first aspect of the present invention refers to the use of maslinic acid, in its widest definition as it was described above, or any of its derivatives for the preparation of a drug for the treatment of pathologies comprising the activation of COX-2.

Another aspect of the present invention refers to the use of maslinic acid or any of its derivatives to prepare a drug which also favours chondrocyte tissue regeneration.

In a preferred embodiment of the invention the pathologies comprising the activation of COX-2 and/or in those which chondrocyte tissue regeneration is produced are selected from the list comprising: arthrosis, rheumatoid arthritis, sciatica, fibromyalgia, hallux valgus or protuberance of the metatarsophalangeal joint (bunion), patellar tendinitis (jumper's knee) or other pathologies responding to NSAIDs specific for COX-2. An even more preferred embodiment comprises its use for the treatment of arthrosis or rheumatoid arthritis.

Another aspect of the present invention refers to a pharmaceutical composition comprising maslinic acid in its widest definition (salts, metabolites, extracts of plants containing it, extract of by-products of industrial processing of the plant or its fruits, or an industrial by-product or a combination of any of these components) or any of its derivates for the treatment of diseases comprising the activation of COX-2. More specifically, useful for the treatment of diseases comprising the activation of the COX-2 enzyme in human or animal chondrocytes through the inhibition of this COX-2.

In a preferred embodiment, said composition also comprises oleanolic acid and/or a pharmaceutically acceptable vehicle. More preferably, the ratio of maslinic acid: oleanolic acid is between 2:1 and 6:1.

That is, maslinic acid, its salts, pharmaceutically acceptable derivatives or their prodrugs, will be formulated in an appropriate pharmaceutical composition, in the therapeutically effective amount, together with one or more vehicles, adjuvants or pharmaceutically acceptable excipients. More particularly, maslinic acid or the mixture of maslinic acid and oleanolic acid is in a proportion between 0.5% and 10% of the composition total, more specifically between 0.5% and 5% and even more specifically between 1 and 3%.

This composition can be administered in different ways, such as for example, but without limiting to, intraperitoneal, oral, buccal, rectal, intramuscular, topical, subcutaneous, intra-articular administration or through inhalation.

More preferably, it is administered topically, subcutaneously or through articular infiltrations. In another preferred embodiment, said composition is presented in a form adapted to topical administration.

In each case the composition will be adapted to the type of administration used, therefore, the composition described above can be presented as tablets, pills, capsules, solutions, emulsions, creams, body milk, ointments, inhalant, sprays, suppositories or any other form of clinically allowed administration and in an amount that is therapeutically effective. More preferably, in the form of a solution, emulsion, cream, body milk or ointment.

In another preferred embodiment, this composition contains maslinic acid or any of its derivatives, obtained from a plant extract of the Olea genus or an industrial by-product of the plant of Olea genus.

By pharmaceutically acceptable derivative we mean any salt, pharmaceutically acceptable or any other compound which after its administration is capable of providing (directly or indirectly) the maslinic acid.

In the sense used in this description, the expression "pharmaceutically acceptable vehicle" refers to those substances, or combination of substances, known in the pharmaceutical sector, used in the production of pharmaceutical forms of administration and including solids or liquids, solvents, tensioactive agents, etc.

Along the description and the claims the word "comprises" and its variants are not intended to exclude other technical characteristics, additives, components or steps. For the experts in the subject, other objects, advantages and characteristics of the invention will be derived partly from the description and partly form the practice of the invention. The following examples and figures will be provided as illustration and are not intended to be limitative of the present invention.

### BRIEF DESCRIPTION OF THE DRAWINGS

**Figure 1** shows the effect of maslinic acid on the production of PGE-2 in human monocytes.
**Figure 2** shows the effect of maslinic acid on the expression of COX-2 in human monocytes.
**Figure 3** shows the effect of maslinic acid on the activity of COX-2 in human monocytes.
**Figure 4** shows the effect of maslinic acid on the production of PGE-2 in SK-N-SH cells.
**Figure 5** shows the effect of maslinic acid on the production of PGE-2 in primary astrocytes.
**Figure 6** shows the effect of maslinic acid on the activity of transcriptional COX-2.
**Figure 7** shows the effect of maslinic acid on the expression of COX-2 in human monocytes.
**Figure 8** shows the effect of maslinic acid on the production of PGE-2 in chondrocytes.
**Figure 9** shows the effect of maslinic acid on the production of IL-6 in chondrocytes.
**Figure 10** shows the effect of maslinic acid on the production of PGE-2 in monocytes.
**Figure 11** shows the effect of maslinic acid on the activity of the IL-6 promoter induced by P + I.
**Figure 12** shows the effect of maslinic acid on the activity of the IL-6 promoter induced by IL-1.
**Figure 13** shows the effect of maslinic acid on the production of isoprostanes in primary monocytes.

### EXAMPLES

Next, the invention will be illustrated.through assays carried out by the inventors, which make clear, among other things, the activity and effectiveness of maslinic acid and show what has been generally described in the sections above.

The compounds, materials and methods used are generally described for all the examples:

### 1. Compounds

Maslinic acid was used in its form of sodium salt, which was dissolved in DMSO (dimethyl sulfoxide) in a stock solution (10 and 50mM). The final maximum concentration of DMSO in cultures was 1% (vol/vol), which does not interfere with the parameters measured.

### 2. Cell Cultures

- Isolation and culture of primary monocytes of peripheral blood. Peripheral blood mononuclear cells (PBMC) coming from healthy adults volunteer donors were isolated by venous blood centrifugation through density gradient with Ficoll-Hypaque. The monocytes were separated through adherence to plastic and later cultured in 24-well plates at a density of 50,000 cells/ml for the enzyme immunoassay (EIA) experiments and in 6-well plates for the immunoblot assays.
- Culture of human primary chondrocytes. Chondrocytes were bought from Promocell (Heidelberg, Germany) and cultured in medium conditioned for this type of cells. The cells were cultured in 24-well plates for the determination of PGE2 and IL-6.
- Culture of rat primary astrocytes. The astrocyte primary cultures were prepared from neonatal rat brains (1 day old). The upper part of the brain was opened and the meninges carefully separated to avoid contamination of the cultures with fibroblasts. The cerebral cortex was isolated and a cellular suspension was obtained, which was cultured in DMEM conditioned for the astrocyte culture. The cultures were expanded in 6-well plates and the production of PGE2 measured in cells after the 3 or 4 pass.
- Culture of cell lines. SK-N-SH (*American Tissue Culture collection*, *HTB 11*) is a cell line derived from a human neuroblastoma and an exponential growth in DMEM supplemented by 2mmol/l L-glutamine, antibiotics and 10% FCS. The cells were cultured at 37°C and in an atmosphere at 5% CO₂.

### 3. Western blots

The cells (primary monocytes or SK-N-SH) were stimulated as indicated in each case and lysis solution in 50µl (20mM Hepes pH 8.0, 10mM KCl, 0.15mM EGTA, 0.15mM EDTA, 0.5mM Na₃VO₄, 5mM NaF, 1mM DTT, leupeptin 1µg/ml, pepstatin 0.5µg/ml, aprotinin 0.5µg/ml, 1mM PMSF y 0.5% NP-40). The protein concentration was determined through the Bradford reagent (Bio-Rad, Richmond, CA, USA) and 30µg of proteins were boiled in Laemmli solution and submitted to electrophoresis in gel of SDS/polyacrylamide at 10%. The proteins separated were transferred to a nitrocellulose membrane (0.5 A, 100V at 4 °C) for 1 hour. The membranes were blocked in TBS containing 0.1% Tween 20 and 5% powdered skimmed milk. The immunodetection of the different proteins was carried out through incubation with the different primary antibodies during two hours, followed by incubation with the corresponding secondary antibodies marked with peroxidase for one hour and a half and developed through the use of the chemiluminescence system ECL (GE Healthcare Life Sciences).

### 4. Transitory transfections and luciferase assays

SK-N-SH cells were transfected with the plasmids COX-2-Luc and IL-6-Luc using the JetPei reagent (Polyplus transfection, France) according to the manufacturer instructions. Both plasmids contain the gene promoters indicated directing the luciferase gene as marker of transcriptional activation. The cells were maintained in culture in normal conditions up to 24 or 48 hours after the transfection depending on the experiment and they were stimulated in the same way in each case. After the stimuli, the cells were lysed in lysis buffer (25mM Tris-phosphate pH 7.8, 8mM MgCl₂, 1 mM DTT, 1 % Triton X-100, and 7 % glycerol) for 15 minutes at room temperature. The luciferase activity in the protein extract was determined through the use of the luminometer Autolumat LB9510 (Berthold) following the system instructions of Luciferase assay from Promega (Madison, WI, USA).

### 5. Determination of PGE₂ production in different cell types and of the COX-2 activity

Chondrocyte, monocyte and astrocyte cultures were stimulated with the corresponding activators (IL-1, LPS or PMA plus lonomicine) in presence or absence of several concentrations of maslinic acid during the indicated times. Later, the supernatants were collected to determine the amount of PGE₂ released by the stimulated cells. PGE₂ was determined using the EIA system of Assay Designs (catalogue number 901-001) following the manufacturer instructions.

In order to determine COX-2 activity, the cells were LPS-stimulated first (10ng/ml) for 24 hours to induce the cellular expression of the COX-2 protein. After that time the cultures were washed and a new free of serum culture medium, maslinic acid (different concentrations) and arachidonic acid (AA) were added for 15 minutes. During this time, the enzymatic activity of COX-2 was measured by its capacity to produce PGE2 from the AA included in the culture.

### 6. Determination of the production of isoprostanes and IL-6 in monocytes

Monocyte cultures were stimulated in the same way as for the production of PGE2 (LPS stimulation for 24 hours) and the amount of 8-iso-prostaglandine F_{2α} (isoprostanes) or IL-6 released by the cells was determined respectively through the systems EIA from Cayman (USA) (catalogue number 516351) or from Pelikine (catalogue number RDI-M1916clb) following the manufacturer instructions.

### EXAMPLE 1

### Maslinic acid (MA) inhibits enzymatic activity of the COX-2 enzyme and the production of PGE₂ in LPS-stimulated monocytes.

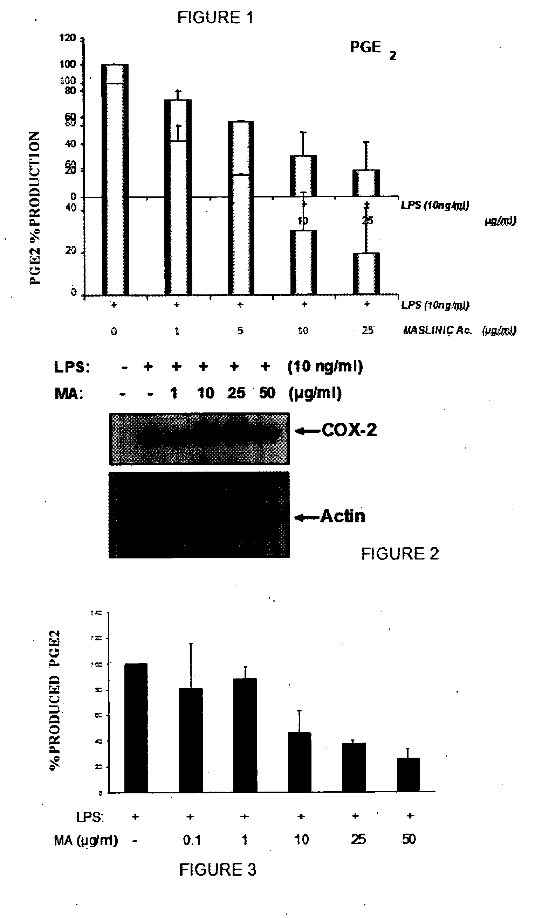

In inflammatory processes monocytes produce numerous pro-inflammatory cytokines such as TNFα, IL-1β and IL-6 and mediators of inflammation such as leukotrienes and prostaglandins, among others. It has been already proven that maslinic acid (MA) inhibited the production of PGE₂ in human monocytes obtained from peripheral blood and LPS-stimulated (FIGURE 1).

In order to identify the action mechanism of MA on the production of PGE₂, a very important aspect to search the cause and be able to predict applications, a series of experiments were carried out intended to study the effect of MA on the transcriptional activity of the COX-2 gene and on the protein expression. First, monocytes in peripheral blood were LPS-stimulated in the presence of growing doses of MA and 12 hours later the cellular properties that were submitted to electrophoresis were extracted to detect the expression of COX-2 through western blots. In figure 2 it can be seen that MA did not inhibit the expression of COX-2 induced by LPS (FIGURE 2).

Vis-à-vis the possibility of MA acting as inhibitor of COX-2 activity, we induced the expression of COX-2 through the treatment of monocytes with LPS for 24 hours. After this time, the cells were washed three times with a medium without serum and incubated for 15 minutes with growing doses of MA. After this time, arachidonic acid was added to the cultures (15µM) for other 15 minutes and the conversion to PGE₂ was measured by EIA. In figure 3 it can be seen that the MA inhibited in a dose-depending manner the enzymatic activity of COX-2 and it explains its effect on the production of PGE₂ in this cell type (FIGURE 3).

### EXAMPLE 2

### Dual effect of maslinic acid (MA) on the production of PGE2 in primary astrocytes and in the SK-N-SH cell line (neuroblastoma)

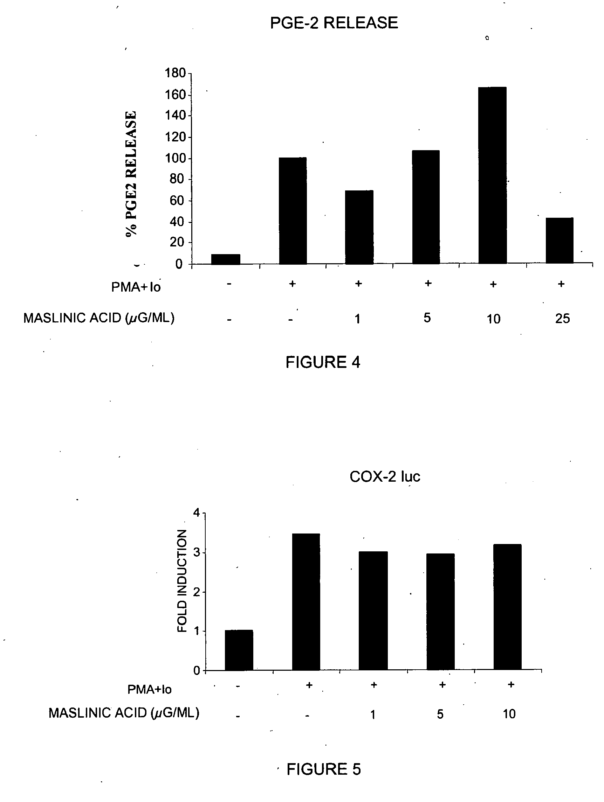

Next, the effect of MA on the transcriptional activity of the promoter of the COX-2 gene and on the expression of the protein in SK-N-SH cells was studies. These cells were transitorily transfected with a plasmid containing the luciferase gene directed by the complete promoter of the COX-2 gene. 24 hours after the transfection the cells were incubated with growing doses of MA and stimulated with PMA plus lomomicine for 6 hours. After this time the luciferase activity was measured in the cellular lyses observing that MA did not affect the transcriptional activity of the COX-2 gene (FIGURE 5).

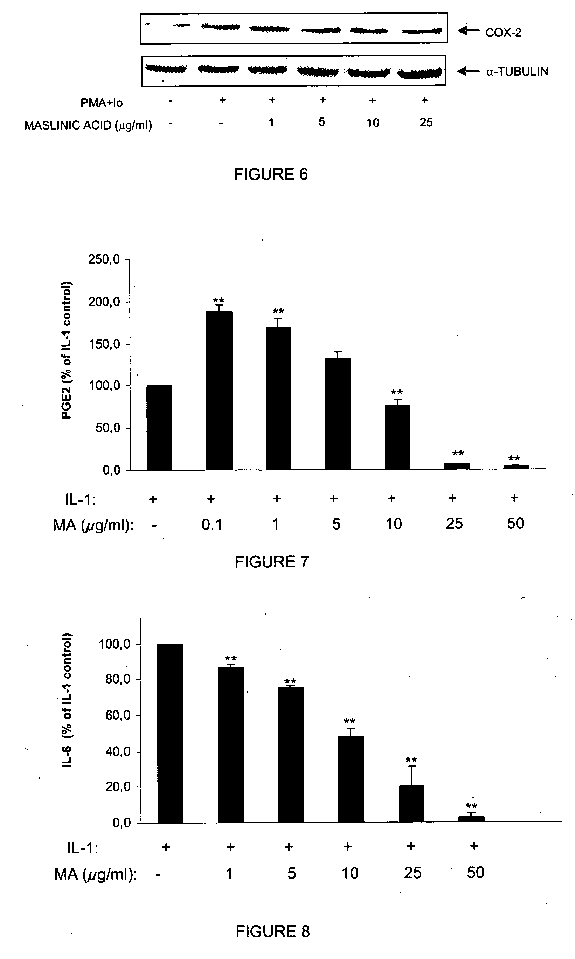

The inducible expression of the COX-2 protein was not affected in SK-N-SH cells by the presence of MA (FIGURE 6).

### EXAMPLE 3

### Effect of maslinic acid (MA) on the production of PGE2 and IL-6 in human chondrocytes stimulated with IL-1

In order to determine if the effect of MA on the production of PGE2 also occurred in another cell type involved in inflammatory phenomena the anti-inflammatory activity of maslinic acid on primary human chondrocytes was determined. The cells were treated with growing doses of MA and later stimulated with IL-1 (10U/ml) to determine the release of PGE₂ and IL-6. In figure 7 (FIGURE 7) it is shown that MA has a dual effect on the production of PGE₂ after the stimulation with IL-1 in chondrocytes. At low doses (0.1 and 1µg/ml) there was an increase in the production of PGE2, while at higher doses (25 and 50µg/ml) a complete inhibition of the release of this prostaglandin induced by IL-1 was observed.

On the contrary, the production of IL-6 was completely inhibited by MA in the same cells and in a manner depending on the concentration (FIGURE 8).

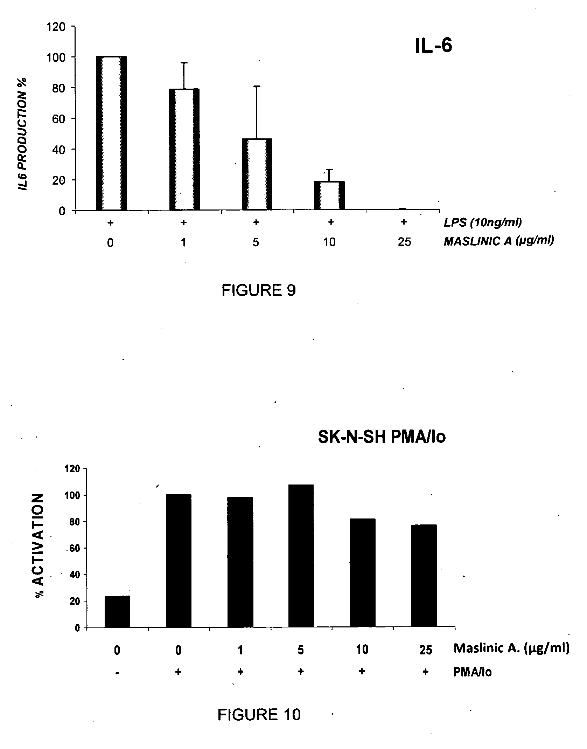

These results totally agree with the results previously found in human monocytes where IL-6 induced by LPS was inhibited by the MA (FIGURE 9).

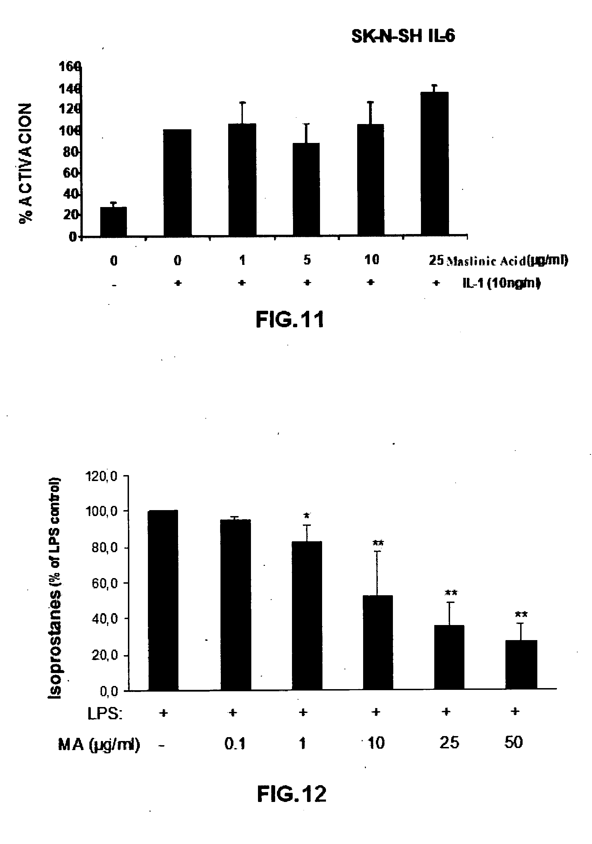

Next, it was studied if the inhibiting effect of maslinic acid on the release of IL-6 was due to a transcriptional effect on the gene codifying said protein. Thus, SK-N-SH cells, which produce IL-6, were transfected with a plasmid containing luciferase gene directed by the complete promoter of IL-6 and stimulated with IL-1 or a combination of PMA plus ionomycin in presence or absence of growing doses of MA and the luciferase activity measured in the cell extracts after 4 hours of treatment. In figures 10 and 11 (FIGURES 10 and 11) there is shown that MA does not inhibit the transcriptional activity of the IL-1 promoter, so the inhibiting activity on the release of IL-6 is surely due to an inhibition at post-transcriptional level.

### EXAMPLE 4

### Effect of maslinic acid (MA) on the production of isoprostanes in LPS-stimulated human monocytes

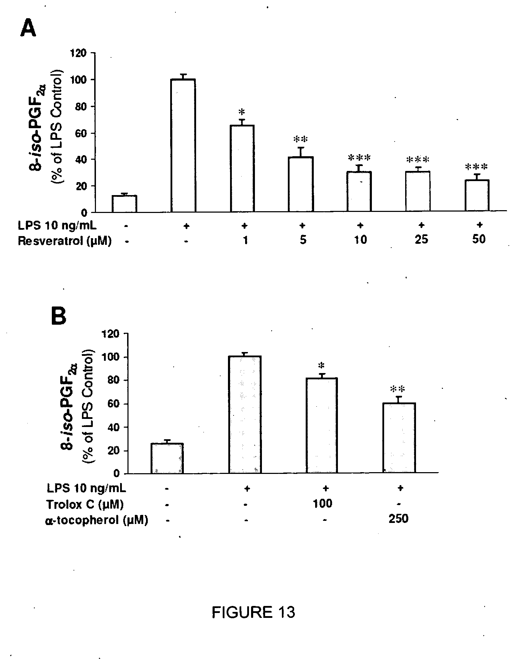

Isoprostanes such as endoperoxide 8-*iso*-prostaglandin F_{2α} (8-*iso*-PGF_{2α}) are metabolites of the oxidation of arachidonic acid and nowadays it is considered one of the best markers of oxidatiye stress. Figure 12 (FIGURE 12) shows that MA inhibits in a concentration depending way the release of 8-*iso*-PGF_{2α} produced in human monocytes of peripheral blood after LPS stimulation. This datum confirms the antioxidant activity of MA previously shown by these authors. The inhibiting power of MA on the production of isoprostanes is higher than that of tocopherol and trolox and is comparable to that of resveratrol (FIGURE 13).

### EXAMPLE 5

Clinical studies were carried out through the topical administration of sodium salt of maslinic acid and mixtures, in variable proportions, of the sodium salts of maslinic acid and oleanolic acid and of maslinic acid and oleanolic acid in variable proportions (from between 70% and 85% of maslinic and between 30% and 15% of oleanolic). In the case of using salts, topical administration was carried out duly distributed in a body milk and in the case of free acids the Beeler base was used for its administration. The body milk contained approximately between 0.5% and 1.5% of the active principle (salts) and the cream contained approximately between 1.5% and 2.5% of the active principle (free acids) as well as extracts of residues of olive crushing enriched in oleanolic acid and maslinic acid.

The sodium salts were obtained through basic treatments with MeONa in methanol or with aqueous solutions of NaOH following usual laboratory procedures.

Some of the results obtained by topical treatments are shown in table I:

**TABLE I**

| **AREA** | **GENDER** | **AGE** | **DIAGNOSIS** | **RESULT** | **OBSERVACIONS** |
|---|---|---|---|---|---|
| KNEES | Male | 61 | ARTHROSIS | POSITIVE | |
| FINGERS | M | 61 | ARTHROSIS | POSITIVE | |
| SPINE | M | 61 | ARTHROSIS | POSITIVE | |
| SHOULDER | M | 80 | ARTHROSIS | POSITIVE | PERMANENT |
| WRISTS | Female | 50 | ARTHROSIS | POSITIVE | PERMANENT |
| SPINE | M | 50 | ARTHROSIS | POSITIVE | |
| KNEES | F | 58 | ARTHROSIS | POSITIVE | |
| FEMUR N. | M | 55 | ARTHROSIS | POSITIVE | |
| FINGERS | F | 55 | ARTHROSIS | POSITIVE | |
| FINGERS | F | 40 | ARTHROSIS | POSITIVE | |
| KNEES | M | 52 | ARTHROSIS | POSITIVE | PERMANENT |
| SHOULDER | M | 53 | ARTHROSIS | POSITIVE | |
| SCIATICA | F | 51 | SCIATICA | POSITIVE | PERMANENT |
| SHOULDER | M | 64 | ARTHROSIS | POSITIVE | |
| COCCYX | F | 50 | ARTHROSIS | POSITIVE | |
| SPINE | F | 53 | SCIATICA | POSITIVE | |
| SHOULDER | F | 92 | ARTHROSIS | POSITIVE | |
| FINGERS | M | 65 | ARTHROSIS | POSITIVE | |
| FEET | F | 61 | ARTHROSIS | POSITIVE | |
| KNEES | F | 63 | ARTHROSIS | NEGATIVE | |
| SHOULDER | M | 63 | ARTHROSIS | POSITIVE | |
| FINGERS | F | 75 | ARTHROSIS | POSITIVE | |
| SPINE | F | 52 | ARTHROSIS | POSITIVE | |
| FINGERS | F | 65 | ARTHROSIS | POSITIVE | |
| KNEES | M | 54 | ARTHROSIS | POSITIVE | |
| SPINE | M | 54 | ARTHROSIS | POSITIVE | |
| SCIATICA | M | 54 | SCIATICA | POSITIVE | PERMANENT |
| FINGERS | F | 63 | ARTHROSIS | POSITIVE | |
| FINGERS | F | 52 | ARTHROSIS | POSITIVE | |
| KNEES | F | 63 | ARTHROSIS | POSITIVE | |
| GENERAL PAIN PAIN | F | 52 | FIBROMYALGIA | POSITIVE | |
| GENERAL PAIN | F | 54 | FIBROMYALGI A | POSITIVE | |
| GENERAL PAIN | F | 48 | FIBROMYALGIA | POSITIVE | |
| GENERAL PAIN | F | 55 | FIBROMYALGIA | POSITIVE | |
| FEET | F | 63 | METATARSOP HALANGEAL JOINT (BUNION) | POSITIVE | |
| KNEES | M | 86 | ARTHROSIS | NEGATIVE | |
| SPINE | F | 67 | LUMBAR ARTHROSIS | POSITIVE | |
| FINGERS | F | 48 | ARTHROSIS | POSITIVE | |
| KNEES | V | 61 | JUMPER SYNDROME | POSITIVE | |
| KNEES | V | 50 | JUMPER SYNDROME | POSITIVE | |
| FINGERS | H | 48 | ARTHROSIS | POSITIVE | |
| FINGERS | H | 35 | ARTHROSIS | POSITIVE | |
| FEMUR N. | V | 54 | ARTHROSIS | POSITIVE | PERMANENT |

Positive result means important reduction of discomfort and considerable increase of flexibility of the joint, waiting between 12 and 24 hours to the following topical treatment, which shows an effective anti-inflammatory effect in the cartilaginous area. In some cases, the disappearance of symptoms has been permanent, which shows a chondrocyte regeneration process. It is surprising that, as regards symptoms, it was achieved in many cases from the first application.

Among more than one hundred patients, only two results were labeled as "negative" which were evaluated specifically. The first one corresponds to a 60-year-old woman with a more than 20-year-old history of arthrosis in knees and feet, and after two months of treatment she informed of a noticeable improvement in her foot discomfort. The second case corresponds to a man over 85 years of age and with serious discomfort in his knees who did not notice any improvement in the treatment.

## Claims

1. Use of maslinic acid or any of its derivatives in the preparation of a medicine for the treatment of pathologies comprising the activation of COX-2.

2. Use of maslinic acid or any of its derivatives in the preparation of a medicine for chondrocyte tissue regeneration.

3. Use of maslinic acid or any of its derivatives according to anyone of claims 1 or 2, in the preparation of a medicine for the treatment of pathologies selected from the list comprising: arthrosis, rheumatoid arthritis, sciatica, fibromyalgia, patellar tendinitis or bunion.

4. A pharmaceutical composition comprising maslinic acid or any of its derivatives, for the treatment of pathologies comprising the activation of COX-2.

5. Pharmaceutical composition according to claim 4, comprising a pharmaceutically acceptable vehicle.

6. Composition according to claims 4 or 5, comprising oleanolic acid.

7. Composition according to claim 6, the ratio between maslinic acid and oleanolic acid being 2:1 and 6:1.

8. Composition according to anyone of claims 4 to 7, in a form adapted to topical administration.

9. Composition according anyone of claims 4 to 8, the maslinic acid or any of its derivatives being obtained from an extract of a plant of the Olea genus.

10. Composition according to anyone of claims 4 to 9, the maslinic acid or any of its derivatives being obtained from an industrial by-product of a plant of the Olea genus.

11. Composition according to anyone of claims 4 to 9, the maslinic acid or any of its derivatives being present in a proportion between 0.5% and 3% of the total of the composition.
